# EUROPEAN PATENT SPECIFICATION

(11) **EP 0 776 641 B1**
(45) Date of publication and mention of the grant of the patent: **01.12.1999**
(21) Application number: 97200334.7
(22) Date of filing: 30.07.1992
(51) Int. Cl.: A61F 2/24, A61B 17/04

(54) **Flexible suture guide and holder**
Biegsame Nähfadenführung und Halterung
Guide de fil de suture flexible et support

(30) Priority: 02.08.1991 US 739925
(43) Date of publication of application: 04.06.1997
(62) Divisional of application: 92916477.0
(73) Proprietor: BAXTER INTERNATIONAL INC., Deerfield, IL 60015-4633 (US)
(72) Inventor: Cosgrove, Delos M., Hunting Valley, Ohio 44022 (US); Nguyen, Than, Huntington Beach, California 92646 (US)
(74) Representative: MacGregor, Gordon

(56) References cited:
- EP-A- 0 257 874
- WO-A-91/01697
- FR-A- 2 617 396
- US-A- 4 185 636
- US-A- 4 489 446
- US-A- 4 492 229
- US-A- 5 011 481

## Description

The present invention relates to a combination of a flexible suture guide releasably attached to a rigid holder that is usable, e.g. for controlling the spacing and placement of surgical sutures. The surgical suture guide can be used to control the dimension and/or shape of a suture line placed during surgical repair of an organ or body part.

During surgical repair of an organ or other body part, the surgeon typically makes an incision to open the organ. Upon closure of the surgical wound, sutures are placed in the various layers of tissue to draw the two edges of the wound together so that the healing process can reform a smooth and competent surface. However, sutures often tear through the tissue if they are subjected to stress, thus damaging the surgical closure of the wound. It would be desirable in many instances to have a means for lending permanent support to strengthen and support the wall of the organ into which the surgical incision has been placed. Alternatively, in many instances it would be preferred to have a biodegradable suture guide.

It is desirable to use a suture guide to aid the surgeon in achieving the desired dimension of the surgical closure and/or to rigidly support the area where the sutures are placed, thus avoiding the danger that the sutures will tear through the tissue or that the suture line will act like a draw string and undesirably bunch up the tissue.

It is desirable that a suture guide be entirely flexible, light weight, and compliant while having sufficient strength to withstand stress placed upon the sutures sewn through and around it. However, an entirely flexible suture guide cannot prevent bunching of the tissue in the draw-string effect described above and thus cannot assure that the suture line and the tissue into which it is placed will maintain any desired dimension, for example, a desired circumference. WO-A-91/01697 discloses a means of temporarily providing rigidity and fixed dimension to the suture guide during the surgery, but rendering the suture guide freely flexible once the surgery has been accomplished.

WO-A-91/01697 thus discloses a suture guide and holder combination comprising: a biocompatible, freely flexible, surgical suture guide of predetermined length; a rigid holder holding the length of the suture guide in a plane around a perimeter of the holder; and at least one thread releasably retaining the suture guide on the holder in a taut fashion.

The present invention provides a suture guide and holder combination, as set out in Claim 1. The pre-characterising part of Claim 1 is based on WO-A-91/01697.

The present invention is distinguished in that the holder has a linear groove, receiving the suture guide.

The suture guide can be formed of either biodegradable or non-biodegradable materials depending upon whether its purpose is to serve as a permanent support to prevent tearing out of the sutures placed through it or whether the suture-supporting function is to be a temporary one. In addition to its function as a post-surgical support for sutures, during surgery when used in combination with the holder disclosed herein, the suture guide serves as a rigid support and template by which the surgeon controls the length of the finished suture line.

In one embodiment, the thread attaches the suture guide to the body surface at least at two points, for example at its extreme ends, by passing at least partially through the suture guide and about the body, i.e. by means of an in and out stitch or stitches.

The thread is provided such that the suture guide can be released from the body once the suture line has been placed by the surgeon without disturbing the sutures sufficiently to cause dislocation or tearing of the sutures through the tissue. A portion of the thread(s) affixing the suture guide to the body can be positioned to be cut by scissors, or the like, to freely release the suture guide from the body. When the thread or threads are cut or otherwise ruptured, the suture guide is freely released from the body.

US-A-4489446 discloses a rigid annuloplasty ring having two arcuate ring portions telescopically engaged to permit adjustment of the ring in accordance with movement of a valve annulus during the cardiac cycle.

### DESCRIPTION OF THE DRAWINGS

The present invention may be better understood and the advantages will become apparent to those skilled in the art by reference to the accompanying drawings, wherein like reference numerals refer to like elements in the several figures, and wherein:
Figure 1 is a perspective exploded view of a flexible suture guide mounted on a rigid holder assembly.
Figure 1A is a top view in partial cross-section of a length of a flexible suture guide.
Figure 1B is a top view in partial cross-section of the flexible suture guide sutured into a ring configuration.
Figure 2 is an exploded view of the guide mount portion and lower part of the handle portion of the holder assembly of Figure 1 without the suture guide.
Figure 3 is a perspective view of a flexible suture guide mounted on the assembled guide mount and lower handle portions seen in Figure 2.
Figure 4 is a cross-sectional view of the assembled guide mount and lower handle portions of Figure 3 along line 4-4.
Figure 5 is a top view of the guide mount seen in Figure 3 with a flexible suture guide tautly secured thereto.
Figure 6 is a perspective view of a guide mount.
Figure 7 is a cross-sectional view of a suture guide having a lenticular cross-sectional shape.
Figure 8 is a side perspective sectional view of a handle assembly.
Figure 9 is a bottom view of the handle extension of Figure 8.
Figure 10 is a top view of the housing of Figure 8.
Figure 11 is a perspective view of another suture guide holder.
Figure 12 is an exploded view of the guide mount portion and lower handle portion of the suture guide holder of Figure 11.
Figure 13 is a top view of the guide mount portion of the suture guide holder of Figure 11.
Figure 14 is a perspective view of a linear suture guide holder in accordance with the invention.
Figure 15 is a perspective view of the suture guide holder of Figure 14 with a suture guide attached.

### DESCRIPTION OF THE PREFERRED EMBODIMENTS

The present invention is generally directed to a holder assembly holding a substantially flexible, implantable suture guide in a substantially taut position for suturing along a suture line having a desired shape or dimension. The suture guide of the preferred embodiment is formed from a freely flexible rib encased within a woven cloth covering. In use, the flexible suture guide is held taut by the holder assembly and in a configuration determined by the shape of the holder assembly while the surgeon uses the support provided by the taut suture guide to evenly place the sutures and to draw the tissue by means of the sutures passed through the suture guide into a suture line having a shape substantially similar to that of the suture guide and holder.

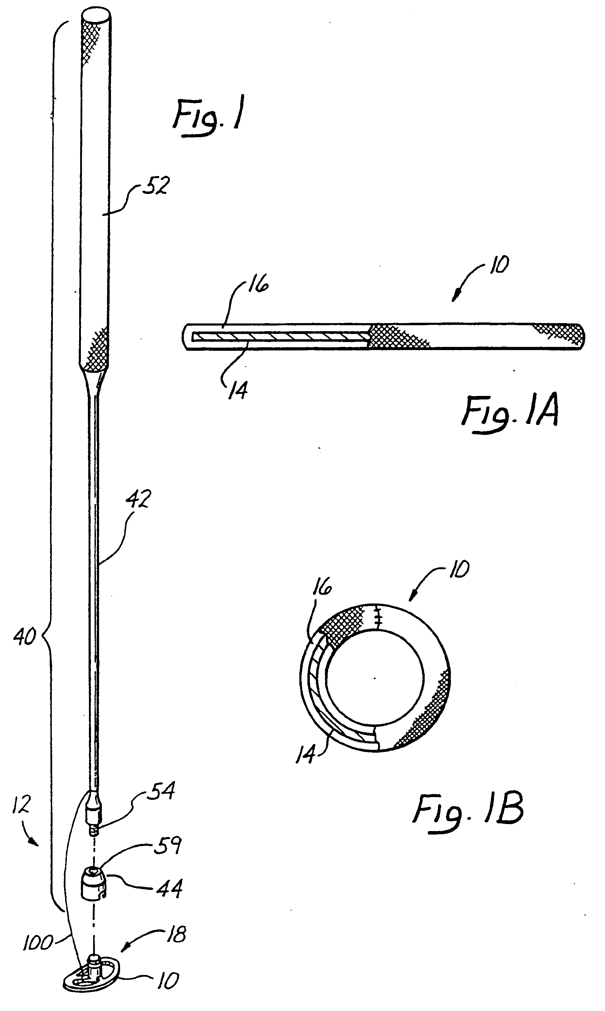

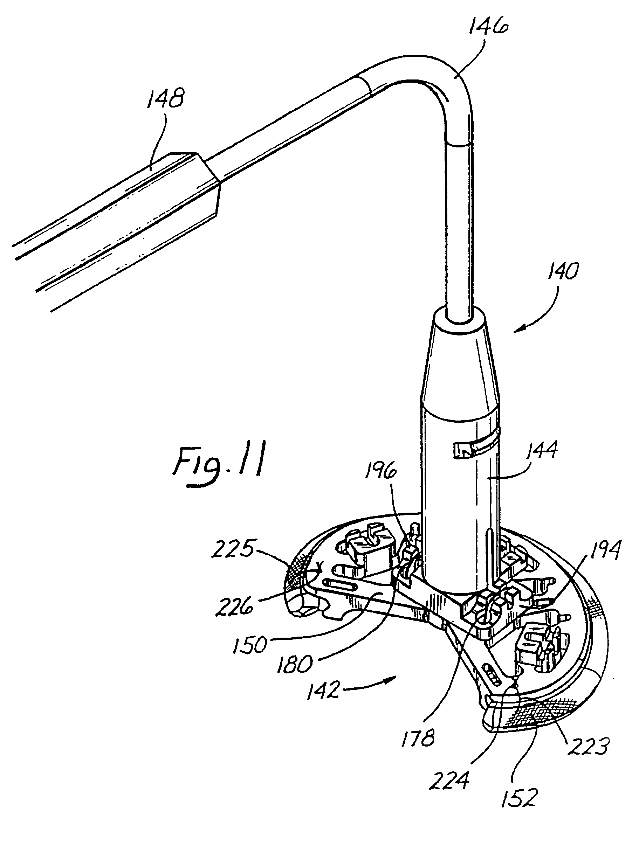

The holder assembly allows the surgeon to properly position the suture guide while the suture guide is used to draw the sutures and associated tissue into the desired configuration during the suturing process. The freely flexible suture guide is given temporary rigidity during surgery by the detachable holder assembly, thus lending precision to the surgeon in controlling the placement and location of the stitches in the suture line. The disclosure with respect to Figures 1 to 13 is not in accordance with the invention as claimed in Claim 1.

Referring now to Figure 1A, the suture guide 10 is an elongate, flexible member of a predetermined dimension. Due to its flexibility, the length of suture guide 10 can be manipulated to assume any desired shape, i.e. circular, C-shaped, straight, curvilinear or a combination of curvilinear and straight segments. In Figure 1B, suture guide 10 is shown as shaped into a ring by suturing the two ends together with sutures 11. As shown in Figure 1A, suture guide 10 comprises a substantially flexible inner rib 14 encased within covering 16.

Rib 14 comprises a flat, rod-like or tubular piece of biocompatible resilient, flexible material, such as mylar or silicone rubber. Rib 14 can also contain a substance opaque to x-rays, for example, about 10 to 15 weight percent, preferably 13 weight percent, of barium sulfate so that the location of the suture guide can be determined in post-operative x-rays. The outer covering 16 is formed from any biocompatible material having sufficient strength to serve as an anchor to sutures without tearing and sufficient flexibility to be formed into a tight covering for rib 14 without restricting flexibility of suture guide 10. Preferably, the outer covering 16 is a woven cloth having a nap to encourage tissue ingrowth, for example a dacron valour. This outer covering 16 is tightly wrapped and sewn about frame 14 so as to completely encase it. The thickness of the outer cloth 16 is sufficient to allow the surgeon to pass a suture therethrough.

Figure 1 shows an exploded view of one embodiment of a holder assembly to which a suture guide is mounted, as seen generally at 12 and 10 respectively. The holder assembly 12 includes a guide mount assembly 18 and handle assembly 40 comprising a handle 42 and housing 44.

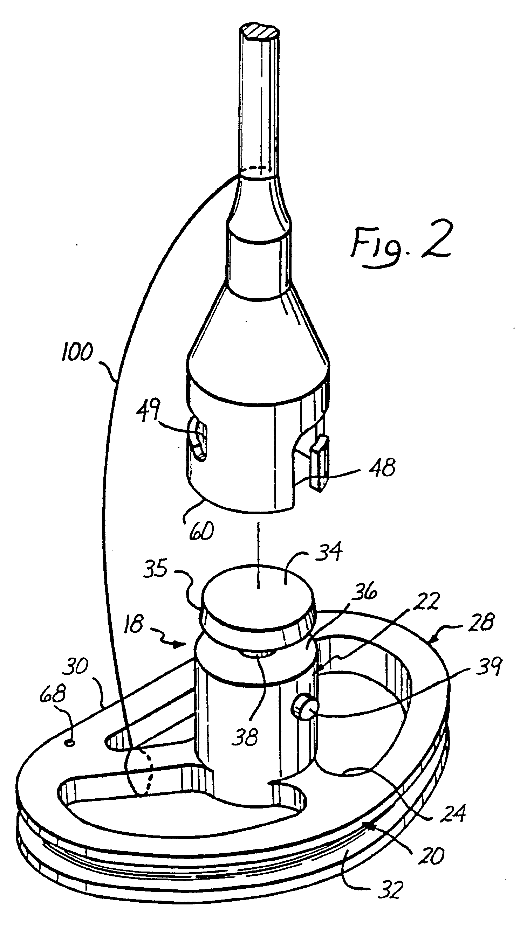

Figures 2 through 5 illustrate in greater detail the guide mount assembly 18 and how the suture guide 10 is mounted thereon. Guide mount assembly 18 includes a guide support 20. For illustrative purposes, the suture guide assembly 18 here shown is one intended for use in plication of a distended heart valve annulus. Therefore facing edge of guide support 20 is generally C-shaped or annular, having a shape and circumferential dimension similar to that the surgeon desires to achieve in the human heart annulus by means of annuloplasty surgery. More particularly, support 20 is generally lenticular, having a C-shaped portion 28, with its ends connected by a straight side 30.

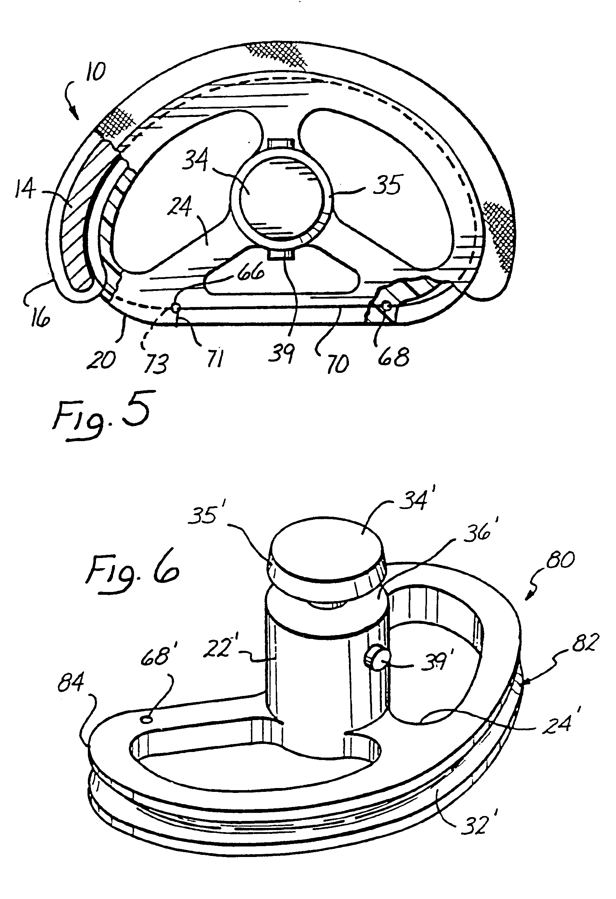

The suture guide 10 is fitted into a groove or trough 32 located about the curved C-shaped portion 28 of the guide support 20 Trough 32 is dimensioned to receive a portion of the suture guide 10, as best seen in Figure 4. The positioning of the suture guide 10 within the trough 32 conforms guide 10 to the shape of the guide support 20 while exposing a substantial portion of the covering 16 outside of the trough 32 to allow the surgeon to pass a suture therethrough.

In the embodiment shown in Figures 2-5, the guide mount assembly 18 also includes a central support hub 22 to which the guide support 20 is attached by a multiplicity of integrally formed spokes, preferably three, one of which is seen at 24. The arrangement of mount assembly 18 including, in this instance, a curved guide support 20 with hub 22 and spokes 24, allows the surgeon to visually observe the heart valve during the suturing process. Central support hub 22 is formed with an annular groove 36. This groove 36 is formed proximate that end 34 of hub 22 opposite guide support 20, and defines a post member 38. That portion of hub 22 remaining on the side of the groove 36 opposite the guide support 20, and hub end 34, includes an inwardly tapering peripheral surface, as seen generally at 35. The hub 22 also includes an open bore 37 through which is fitted a cylindrical plug 39. The plug 39 is dimensioned to extend out from both sides of the bore 37. The purpose of tapered surface 35, and the plug 39 will be described hereinafter.

As is further seen in Figure 1, the handle assembly 40 includes an elongated handle 42 having end 54 mounted to housing 44. While housing 44 may be integrally formed at the end 54 of the post 42, preferably end 54 is formed with outwardly facing threads that threadably mate with threads formed along a surface of an opening 59 formed in the top of the housing 44. The opposite end of post 42 is formed with an external etched surface 52 to assist the surgeon in gripping post 42. In another embodiment, end 54 of post 42 and opening 59 of housing 44 can be formed so that end 54 can be press fit into opening 59.

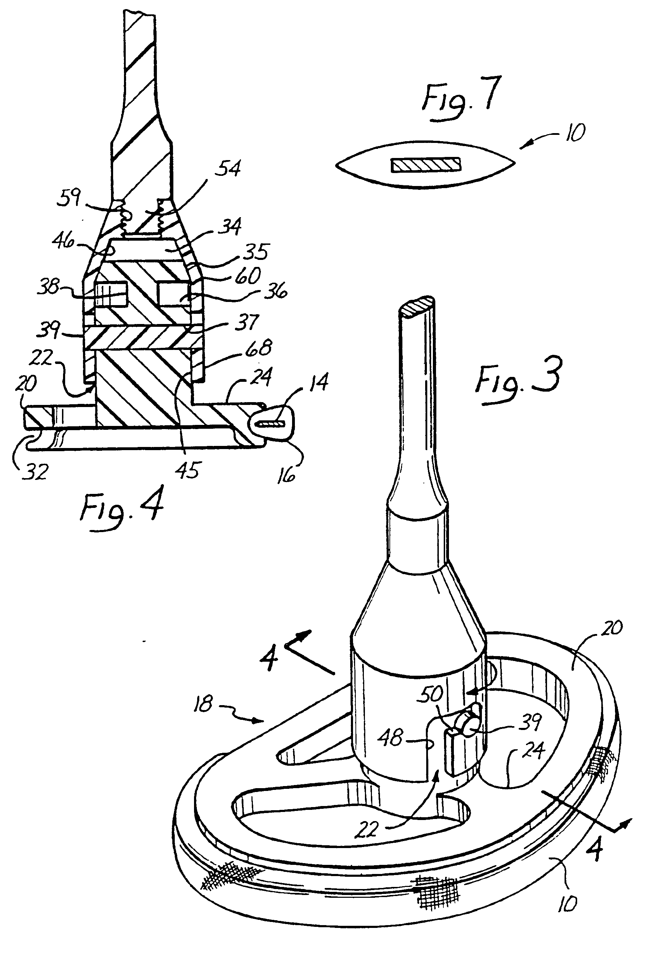

Housing 44 is a thimble-shaped structure having a circular wall 60 defining a cavity 46. As seen better in Figure 4, cavity 46 is open at one side, seen generally as opening 45. The inner surface of the circular wall 60 inwardly converges a short distance from the opening 45. The cavity 46 is generally wide enough at the open side 45 to snugly receive hub 22, but the plug 39 extends sufficiently outward from hub 22 to prevent passage through open side 45 into cavity 46. Wall 60 is formed with two J-shaped notches, seen at 48 and 49 in Figures 2 and 3. These J-shaped notches 48 and 49 are formed and positioned to respectively receive the ends of the plug 39 extending outward from the hub 22. The shape of the notches 48 and 49 defines a landing 50 between the long and short legs of each notch.

Handle assembly 40 is coupled to the guide mount assembly 18 by inserting end 34 of the hub 22 into the cavity 46, with one of the outwardly extending ends of the plug 39 passing through a respective one of the J-shaped notches 48 and 49. The tapered surface 35 of the hub 22 engages the inwardly tapering surface of the wall 60. This causes a slight compression of the hub end 34, resulting in a spring force. The spring force acts to restrain the movement of the outwardly extending ends of the plug 39 through the larger legs of the J-shaped notches 48 and 49. Additional exertion moves the ends of plug 39 through the larger legs of J-shaped notches 48 and 49, with rotation of handle 40 passing the outward ends of plug 39 across the landings 50 and into the smaller leg of the J-shaped notches 48 and 49.

The spring force established by the slight compression of the hub end 34 maintains the coupling between housing 44 and guide mount assembly 18. The handle 40 is decoupled from the guide mount assembly 18 by reversing the described procedure.

One embodiment of the means for releasably attaching suture guide 10 to guide support 20 of guide mount assembly 18 is seen in Figure 5. Guide support 20 is formed with two apertures 66 and 68 extending through guide support 20 and communicating with groove 32. The exact positioning of apertures 66 and 68 is not critical. As illustrated, apertures 66 and 68 are formed along the straight portion of guide support 20, at a location proximate two of the spokes 24.

One end 71 of a cord or suture thread 70 is passed through one of the apertures, as illustrated hole 66, and tied off on guide support 20. The other end 73 of suture 70 is passed through the body of suture guide 10 from one end to the other. This end 73 is then passed first through hole 68 and then through and tied off at hole 66. After suture guide 10 is sutured into position during surgery, i.e., about the valve annulus, that portion of the suture 70 between apertures 66 and 68 is snipped or cut in two. Suture 70 passes out of suture guide 10 by withdrawing the handle assembly 12.

In accordance with another embodiment (not shown), the first end 71 is tied off at hole 66, with the second end 73 passed first through one end of the suture guide 10, and then brought back across and passed through the other end of suture guide 10, through hole 68 and again tied off at hole 66. Removal of suture 70 is accomplished by snipping the suture in two at any point between the two holes and withdrawing it.

An alternative embodiment of the guide mount assembly 80 as seen in Figure 6 includes a guide support 82 having an open C-shaped side 84 but no straight side joining the ends of the C. Except for the stated difference in shape of the guide support 82, guide mount assembly 80 in Figure 6 includes elements similar to those described for the suture guide of Figure 5 (as is indicated by the prime of the previously provided element number), and will be described in no further detail herein. In this embodiment of guide mount assembly 80, the means for releasably attaching the suture guide to the guide support is a suture (not shown) positioned by tying off as described above across an open space between holes 68'and 66' (not shown).

The handle assembly 40 is tethered to the guide mount assembly 18. As seen in Figure 1, this tethering is performed by connecting one end of a lanyard, seen generally at 100, to the handle assembly 40 and the other end of the lanyard 100 to the guide support 20, for instance to one of spokes 24. Lanyard 100 allows a surgeon to detach the handle assembly 40 from the guide support 20 during the suturing procedure to get a clearer view of the surgical site. By tethering handle 40 to the guide mount assembly 18, the risk of the surgeon leaving the guide support 20 in the patient after completion of the surgical procedure is greatly reduced. Lanyard 100 also allows the surgeon to easily remove the guide support 20 after the handle has been detached.

In a preferred embodiment, a handle assembly 40 is modified to house a spool of suture or string that acts like a tether for the guide mount assembly. The tether is attached at opposite ends to the handle assembly and the guide mount assembly respectively and automatically spools out of the handle assembly when the handle is disconnected from the guide mount assembly.

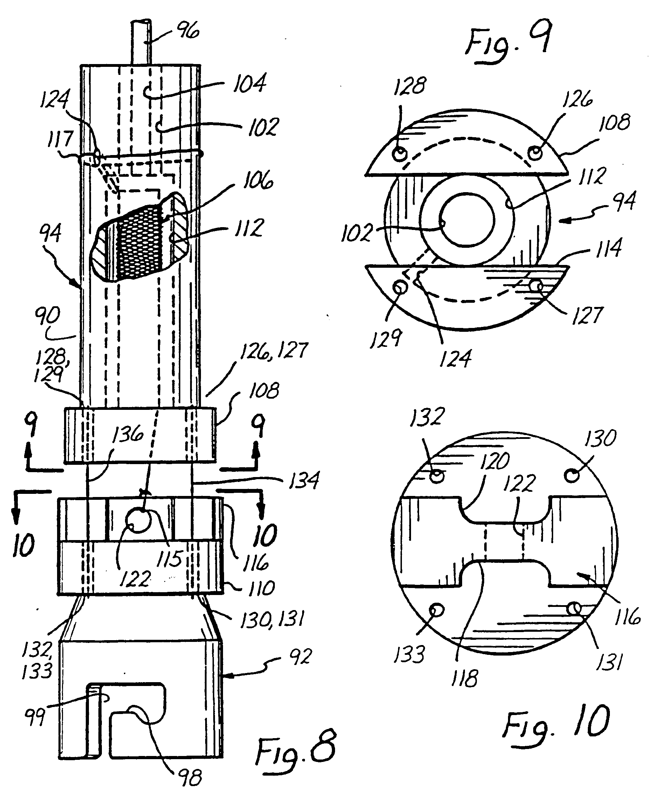

This preferred embodiment is better seen in the several Figures 8 through 10. The lower portion of a handle assembly in accordance with this embodiment is seen in Figure 8 at 90. Handle assembly 90 includes a housing 92, a handle extension 94, and a handle post 96.

Housing 92 includes a pair of opposing J-shaped notches 98 and 99 that function similarly to the J-shaped notches 48 and 49 described above. The handle extension 94 is fastened to the lower end of the handle post 96 in any suitable manner. As shown, the handle extension 94 includes at one end a bore 102 for receiving the lower end 104 of the handle post 96. End 104 of the handle post may be held in bore 102 by welding, stamping, or by providing the respective members with interlocking threaded surfaces. Accordingly, neither of these structures of the handle assembly 90 will be discussed in any greater detail.

The main distinction to the previously described embodiment is that the handle assembly 90 is formed to carry a spool of suture, seen generally at 106. This suture spool 106 is housed in a bore 112 formed in the handle extension 94. Handle extension 94 and housing 92 are formed to releasably fit together. Handle extension 94 and housing 92 include mating collars 108 and 110, respectively. Collar 108 is formed with a groove 114 that receives a tongue 116 extending upward from collar 110. Tongue 116 is formed with a central aperture 122, and two opposing cut-aways 118 and 120 that extend out in opposite directions from this aperture 122.

Each of the collars 108 and 110 possesses four apertures. Apertures 126-129 of collar 108 align with apertures 130-133 of collar 110 when the handle extension 94 and housing 92 are fitted together.

Suture spool 106 comprises a length of suture wound into a cylindrical configuration along lower end 104 of handle post 96, which fits into bore 112. The opposite ends of this suture length are tied to the tongue 116 and the handle extension 94. One end of the suture is drawn through the central aperture of 122 and tied to tongue 116, as seen at 115. The opposite end of the suture is drawn through an opening 124 extending from the bore 112 through the handle extension 94 and is tied around the handle extension 94, as seen at 117. It should be noted that for the purpose of this invention, the meaning of the term "suture" shall include any cord, string or filamentous material useful for tethering the housing 92 to the handle extension 94.

Handle extension 94 and housing 92 are fitted together by placing the tongue 116 into the groove 114. Sutures are run through aligned apertures to hold the handle extension 94 and housing 92 together. For example, one suture 134 is passed through apertures 126 and 127 of handle extension 94 and apertures 130 and 131 of housing 92, while a second suture 136 is passed through apertures 128 and 129 of handle extension 94 and apertures 132 and 133 of housing 92.

The handle assembly 90 of this embodiment is coupled to the guide mount assembly 18 as stated above. The handle post 96 is removed from the housing 92 by cutting the sutures 134 and 136 and pulling the handle extension 94 away from the housing 92. Pulling away the handle post 96 unravels the suture spool 106. After the suture guide is sutured into position along the suture line, i.e., about a heart valve annulus, the suture(s) holding the guide mount assembly to the suture guide is cut. The guide mount assembly is then removed by pulling on the handle post 96.

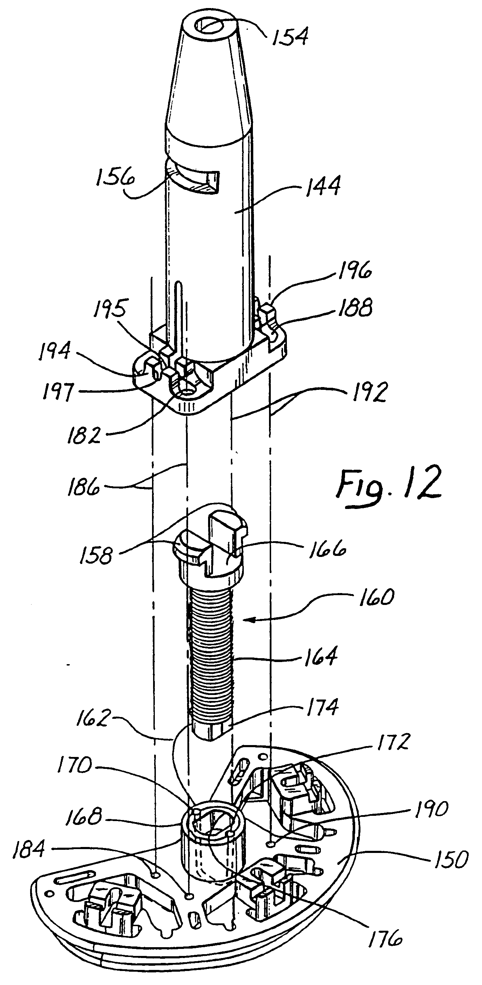

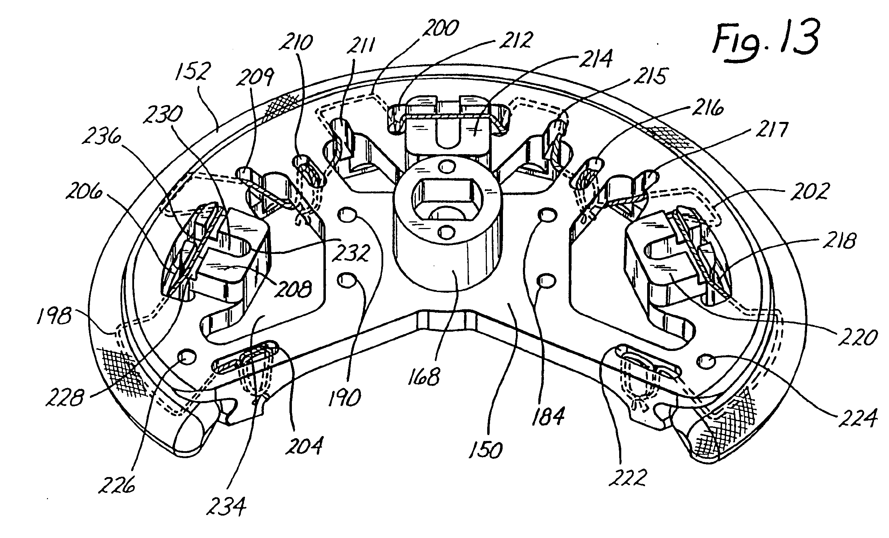

In the embodiment shown in Figures 11-13, handle assembly 140 is also modified to house a spool of suture or string that acts like a tether for the guide mount assembly 142. Referring to Figure 11, handle assembly 140, includes housing 144, handle post 146, and an enlarged handle portion 148. Handle post 146 is preferably made of a malleable metal or other material that allows the surgeon to bend the handle to the desired angle while using the suture guide holder assembly. The enlarged handle portion 148 allows the surgeon to grip the handle more easily and also makes it easier for the surgeon to maneuver the suture guide holder into the surgery site. Housing 144 is releasably attached to guide mount 150 as will be described in more detail with reference to Figure 12. Suture guide 152 is releasably attached to guide mount 150 by threads or sutures (not shown) in a manner which will be described with reference to Figure 13.

Referring to Figure 12, housing 144 includes bore 154 for receiving handle post 146. The end of handle post 146 may be held in bore 154 by a press fit or friction fit, by welding, or by providing the respective members with interlocking threaded surfaces. Housing 144 also includes a pair of opposing slots 156 for receiving dog ears 158 of the suture spool 160. Suture spool 160 includes a length of suture or thread 162 wound into a cylindrical configuration along spindle post 164. One end of the suture 162 is tied to an aperture (not shown) in upper end 166 of suture spool 160. The other end of suture 162 is affixed to hub 168 of guide mount 150. Specifically, suture 162 passes down through aperture 170, up through aperture 172, and is tied off at aperture 172. The lower end of spindle post 164 has a pair of opposing notches 174 formed therein which are sized to be received by bore 176 of hub 168. Spindle post 164, therefore, is press fit or friction fit into bore 176.

Suture spool 160 is housed within the interior cavity (not shown) of housing 144 and is held in place when dog ears 158 snap fit into opposing slots 156. Housing 144 with suture spool 160 in place is then releasably attached to guide mount 150 by sutures or threads 178 and 180 shown in Figure 11. Suture 180 passes through a pair of apertures 182 in housing 144 and a pair of apertures 184 in guide mount 150 as illustrated by dotted lines 186 in Figure 12. Suture 178 passes through a pair of apertures 188 in housing 144 and a pair of apertures 190 in guide mount 150 as shown by dotted lines 192 in Figure 12.

Once the suture guide and guide mount assembly has been placed at the surgery site, she surgeon can remove the handle if desired by cutting sutures or threads 178 and 180 at the location of the cutting guides 194 and 196 shown in Figures 11 and 12. Cutting guides 194 and 196 consist of a raised platform with a shallow groove 195 formed therein through which the suture passes and a deeper groove 197 formed in the platform perpendicular to the shallow groove through which scissors or other cutting tools can be inserted to clip or cut the suture at that location. When the sutures are cut and the handle is removed, spool 160 remains within housing 144 and suture 162 remains attached to hub 168. As the handle is pulled away from the guide mount, the suture or thread spools off spindle post 164 thereby providing a tether for removal of the guide mount after the suture guide has been detached from the guide mount and the surgery has been completed.

Referring to Figure 13, suture guide 152 is releasably attached to guide mount 150 by sutures or threads 198, 200 and 202. Suture 198 is tied off at aperture 204 and then passes through one end of the suture guide and up through aperture 206 over cutting guide 208 down through aperture 206 again, through suture guide 152, through aperture 209, then up through aperture 210 where the other end of suture 198 is tied off. Suture 200 is at one end tied off through aperture 210 and then passes under the guide mount 150 through aperture 211, through suture guide 152 and up through aperture 212, across cutting guide 214 and back down through aperture 212. Suture 200 then passes through suture guide 152 again, through aperture 215 and up through aperture 216 where it is tied off. Finally, suture 202 is tied off at one end at aperture 216 and passes under suture guide 150 through aperture 217, through suture guide 152, up through aperture 218, across cutting guide 220, down through aperture 218 again where it passes through suture guide 152 and up through aperture 222 where it is tied off.

Apertures 224 and 226 disposed in guide mount 150 at opposite ends of the suture guide 152 are used to temporarily attach each end of suture guide 152 to each end of the guide mount 150 to hold the suture guide in place during the process of threading sutures 198, 200 and 202 through the apertures of the guide mount and the suture guide. Once the threading of sutures 198, 200 and 202 is complete, the sutures at 224 and 226 are then removed. The sutures at 224 and 226 are shown for illustration purposes in Figure 11 at 223 and 225.

Referring to Figure 13, cutting guides 208, 214 and 220 consist of a raised platform with a shallow groove 228 formed therein through which the suture passes and a deeper groove 230 formed in the platform perpendicular to the shallow groove through which a cutting tool may pass in order to cut the suture at the location lying over the deeper groove. The deeper groove is closed at one end by a stop 232 so that the tip of the cutting tool or scissors cannot pass beyond that point. This stop prevents the cutting tool from dipping down into the open space 234 between the spokes of guide mount 150 and accidentally cutting the tissue of the patient.

When the surgeon is ready to release the suture guide from the suture guide mount 150 he merely snips the sutures 198, 200 and 202 by passing the cutting tool into the cutting groove of the cutting guides. When the sutures have been snipped at all three locations, the guide mount can be retrieved by pulling on the tether or otherwise removing it and sutures 198, 200 and 202 are removed with the guide mount 150 since they are tied off on the guide mount.

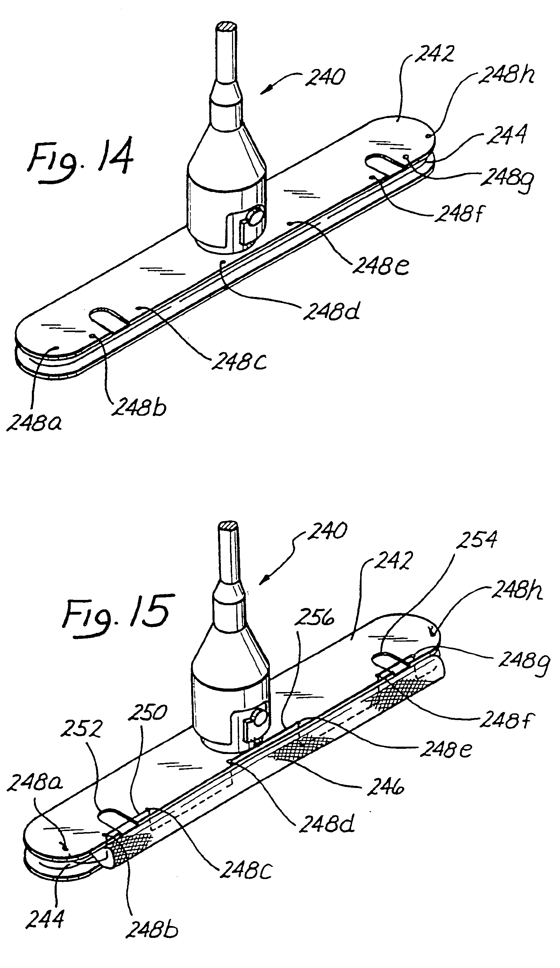

Referring to Figures 14 and 15, there is shown, in accordance with the present invention, a linear suture holder for placing a linear suture guide.

Referring to Figure 14, the linear suture guide holder has a detachable handle 240 of the type shown in Figure 2 and linear- shaped guide mount 242. However, the handle embodiment with the tether illustrated in Figures 8 and 12 could also be used. Guide mount 242 has a linear groove or trough 244 into which suture guide 246 is fitted as shown in Figure 15. Apertures 248 formed in guide mount 242 are used to suture the suture guide to the guide mount as shown in Figure 15. Guide mount 242 also includes cutting guides 252 and 254 at each end of the guide mount.

Suture guide 246 is tautly secured to the linear guide mount 242 by suture 250. One end of suture 250 is tied off at aperture 248a, passes through suture guide 246 up through aperture 248b cross cutting guide 252 down through aperture 248c through suture guide 246 up through aperture 248d where it is tied to a second length of suture 256. Suture 256 is threaded down through aperture 248e through suture guide 246 up through aperture 248f across cutting guide 254 down through aperture 248g through suture guide 246 and up through aperture 248h where it is tied off. Thus, as in previous embodiments, when the surgeon is ready to release the suture guide from the suture guide holder, he merely inserts the cutting tool in the cutting grooves 252 and 254 and cuts sutures 250 and 256 at that location. Suture 250 and 256 are then removed with the suture guide mount 242. The linear suture guide shown in Figures 14 and 15 would be used for any surgical procedure in which the incision is a substantially straight line. The suture guide mount 242 can be any desired length and the suture guide 246 can extend the full length of the suture guide mount 242 as shown in Figure 15 or could be of a shorter length and sutured to just a portion of suture guide mount 242. If the surgeon desires a suture guide with a hook or curved end, the suture guide could extend around the edge of suture guide mount 242 to provide one or two curved or hooked ends.

## Claims

1. A suture guide and holder assembly comprising a biocompatible, freely flexible, surgical suture guide (246) of predetermined length, a rigid holder (242) holding the suture guide and at least one thread (250) releasably retaining the suture guide (246) in a taut fashion,
characterised in that the holder (242) has a linear groove (244), receiving the suture guide (246).

2. An assembly according to Claim 1, including a thread guide (252) receiving the thread to facilitate cutting of the thread to release the suture guide from the holder.

3. An assembly according to Claim 2, including a planar member having a rebate, defining the thread guide (252) and overlying the groove (244).

4. An assembly according to Claim 3, wherein the planar member has apertures (248) receiving the thread (250) and overlying the groove (244).

5. An assembly according to Claim 2, 3 or 4, including a pair of said thread guides (252) spaced along the groove.

6. An assembly according to any preceding claim, wherein the holder (242) has a curved groove portion at an end of said groove (244), with the suture guide (246) having an end portion received in the curved groove portion.

7. An assembly according to any preceding claim, including a handle (240) detachably mounted on the holder (242).

8. An assembly according to Claim 7, including tethering means (100,160-164) tethering the handle (240) to the holder (242).

9. An assembly according to Claim 8, wherein the tethering means comprises a lanyard (100) with one end connected to the holder (242) and the opposite end connected to the handle (240).

10. An assembly according to Claim 8, wherein the tethering means comprises a spool (160) of filamentous material (162,164) having one end connected to the holder (242) and the opposite end connected to the handle (240), the spool being housed within a cavity in the handle.

## Patentansprüche

1. Führungs- und Halteanordnung für Nahtmaterial, die folgendes aufweist
- eine biokompatible, frei biegsame Führung (246) für chirurgisches Nahtmaterial mit vorgegebener Länge,
- einen steifen Halter (242) zum Halten der Nahtmaterialführung und
- mindestens einen Faden (250), der die Nahtmaterialführung (246) in eng anliegender Weise lösbar hält,
dadurch gekennzeichnet,
daß der Halter (242) eine lineare Nut (244) aufweist, die die Nahtmaterialführung (246) aufnimmt.

2. Anordnung nach Anspruch 1,
die eine Fadenführung (252) aufweist, welche den Faden aufnimmt, um das Schneiden des Fadens zu erleichtern, um die Nahtmaterialführung von dem Halter zu lösen.

3. Anordnung nach Anspruch 2,
die ein ebenes Teil mit einer Aussparung aufweist, welche die Fadenführung (252) bildet und über der Nut (244) liegt.

4. Anordnung nach Anspruch 3,
wobei das ebene Teil Öffnungen (248) aufweist, welche den Faden (250) aufnehmen und über der Nut (244) liegen.

5. Anordnung nach Anspruch 2, 3 oder 4,
die ein Paar von Fadenführungen (252) aufweist, die längs der Nut beabstandet sind.

6. Anordnung nach einem der vorherigen Ansprüche, wobei der Halter (242) einen gekrümmten Nutbereich an einem Ende der Nut (244) aufweist, wobei die Nahtmaterialführung (246) einen Endbereich besitzt, der in dem gekrümmten Nutbereich aufgenommen ist.

7. Anordnung nach einem der vorherigen Ansprüche, die eine Handhabe (240) besitzt, welche lösbar an dem Halter (242) angebracht ist.

8. Anordnung nach Anspruch 7,
die eine Anbindeeinrichtung (100, 160-164) aufweist, welche die Handhabe (240) an dem Halter (242) anbindet.

9. Anordnung nach Anspruch 8,
wobei die Anbindeeinrichtung einen Abzugsfaden (100) aufweist, der mit dem einen Ende mit dem Halter (242) und mit dem gegenüberliegenden Ende mit der Handhabe (240) verbunden ist.

10. Anordnung nach Anspruch 8,
wobei die Anbindeeinrichtung eine Spule (160) aus fadenförmigem Material (162, 164) aufweist, die mit dem einen Ende mit dem Halter (242) und mit dem gegenüberliegenden Ende mit der Handhabe (240) verbunden ist, wobei die Spule innerhalb eines Hohlraumes in der Handhabe untergebracht ist.

## Revendications

1. Ensemble de guide de suture et de support comprenant un guide de suture chirurgicale (246) librement flexible, biocompatible, de longueur prédéterminée, un support rigide (242) tenant le guide de suture et au moins un fil (250) retenant de façon libérable le guide de suture (246) de manière tendue, caractérisé en ce que le support (242) a une rainure linéaire (244) qui reçoit le guide de suture (246).

2. Ensemble selon la revendication 1, incluant un guide-fil (252) recevant le fil pour faciliter la section du fil afin de relâcher le guide de suture du support.

3. Ensemble selon la revendication 2, incluant un élément plan ayant une feuillure, définissant le guide-fil (252) et surplombant la rainure (244).

4. Ensemble selon la revendication 3, dans lequel l'élément plan a des ouvertures (248) recevant le fil et surplombant la rainure (244).

5. Ensemble selon la revendication 2, 3, ou 4, incluant une paire dedits guide-fil (252) espacés le long de la rainure.

6. Ensemble selon l'une quelconque des revendications précédentes, dans lequel le support (242) a une partie de rainure courbe à une extrémité de ladite rainure (244), le guide de suture (246) ayant une partie d'extrémité reçue dans ladite partie de rainure courbe.

7. Ensemble selon l'une quelconque des revendications précédentes, incluant une poignée (240) montée de manière détachable sur le support (242).

8. Ensemble selon la revendication 7, incluant des moyens d'attache en laisse (100, 160-164) attachant la poignée (240) au support (242).

9. Ensemble selon la revendication 8, dans lequel les moyens d'attache comprend un cordon (100) dont une extrémité est liée au support (242) et l'extrémité opposée est liée à la poignée (240).

10. Ensemble selon la revendication 8, dans lequel les moyens d'attache comprennent une bobine (160) de matière filamentaire (162, 164) dont une extrémité est liée au support (242) et l'extrémité opposée est liée à la poignée (240), la bobine étant logée dans une cavité formée dans la poignée.
